# EUROPÄISCHE PATENTSCHRIFT

(11) **EP 1 012 307 B1**
(45) Veröffentlichungstag und Bekanntmachung des Hinweises auf die Patenterteilung: **04.05.2005**
(21) Anmeldenummer: 98945214.9
(22) Anmeldetag: 12.08.1998
(51) Int. Cl.: C12N 15/57, C12N 9/50, C12N 9/64, C12N 9/76, C12P 21/06

(54) **AUTOKATALYTISCH AKTIVIERBARE ZYMOGENE VORSTUFEN VON PROTEASEN UND DEREN VERWENDUNG**
ZYMOGENIC PROTEASE PRECURSORS THAT CAN BE AUTOCATALYTICALLY ACTIVATED AND THEIR USE
PRECURSEURS ZYMOGENES DE PROTEASES ACTIVABLES PAR VOIE AUTOCATALYTIQUE ET LEUR UTILISATION

(30) Priorität: 22.08.1997 EP 97114513; 15.10.1997 EP 97117816
(43) Veröffentlichungstag der Anmeldung: 28.06.2000
(73) Patentinhaber: Roche Diagnostics GmbH, 68305 Mannheim (DE)
(72) Erfinder: KOPETZKI, Erhard, D-82377 Penzberg (DE); HOPFNER, Karl-Peter, D-81673 München (DE); BODE, Wolfram, D-82131 Gauting (DE); HUBER, Robert, D-82110 Germering (DE)
(86) Internationale Anmeldenummer: PCT/EP1998/005094
(87) Internationale Veröffentlichungsnummer: WO 1999/010503

(56) Entgegenhaltungen:
- EP-A- 0 319 944
- EP-A- 0 495 398
- EP-A- 0 597 681
- WO-A-96/17943
- WO-A-97/00316
- BRODRICK J.W. ET AL.: "Human cationic trypsinogen" JOURNAL OF BIOLOGICAL CHEMISTRY, Bd. 253, Nr. 8, 25. April 1978, Seiten 2732-2736, XP002055238

## Beschreibung

Gegenstand der Erfindung sind zymogene Vorstufen von Serinproteasen, die aufgrund einer neu in das Molekül eingeführten Proteasespaltstelle autokatalytisch aktivierbar sind, und deren Verfahren zur Herstellung und Vewendung.

Spezifisch spaltende Proteasen spielen eine bedeutende Rolle in der Biotechnologie bei der rekombinanten Herstellung von Peptidhormonen (Übersicht Peptidhormone, siehe: Bristow, A.F.: In: Hider, R.C.; Barlow, D., eds. Polypeptides and Protein Drugs. Production, Characterization and Formulation. London, Ellis Horwood, pp. 54-69 (1991)) wie z.B. Insulin (Jonasson, P. et al., Eur. J. Biochem. 236 (1996) 656-661; WO 96/20724), den Insulin-ähnlichen Wachstumsfaktoren IGF-I und IGF-II (Nilsson, B. et al., Methods Enzymol. 185 (1991) 3-16; Forsber, G. et al., J. Prot. Chem. 11 (1992) 201-211), Relaxin, Parathyroidhormon (PTH) und Parathyroidhormon Peptidfragmenten (Forsberg, G. et.al., J. Prot. Chem. 10 (1991) 517-526; WO 97/18314), Calcitonin (EP-A 0 408 764), Glucagon (EP-A 0 189 998), Glucagon-ähnlichen Peptiden (GLP; WO 96/17942), natriuretischen Peptiden (WO 97/11186), "growth hormon releasing factor" (GRF; WO 96/17942) und Urogastron (Brewer, S.J. und Sassenfeld, H.M., Trends in Biotechnology 3 (1985) 119-122; EP-A 0 089 626) und z.B. N-terminal Initiatormethionin-freien Wachstumsfaktoren und Cytokinen (Übersicht Wachstumsfaktoren und Cytokine, siehe: Wang, E.A., TIBTECH 11 (1993) 379-383; Meyer-Ingold, W., TIBTECH 11 (1993) 387-392) wie z.B. IL-1-β, IL-2, GM-CSF (Miller, C.G. et al., Proc. Natl. Acad. Sci. USA 84 (1987) 2718-2722), G-CSF (EP-B 0 513 073). In der Medizin werden spezifisch spaltende Proteasen z.B. zur Behandlung der Hämophilie B (Faktor IX und Faktor VII), zur Clotauflösung, z.B. nach Herzinfarkt (tPA, "tissue type plasminogen activator" und Streptokinase) und Thrombin (US Patent 5,432,062) zur Förderung der Blutgerinnung bei der Wundbehandlung eingesetzt.

Hinsichtlich Substratspezifität unterscheidet man zwischen Proteasen, die selektiv N- oder C-terminal von einer Aminosäure, wie z.B. LysC Endoprotease nach Lysin, oder selektiv von 2 bestimmten Aminosäuren, wie z.B. Trypsin nach Lysin und Arginin, spalten. Daneben gibt es die sogenannten Restriktionsproteasen (Carter, P.: In: Ladisch, M.R.: Willson, R.C.; Painton, CC.; Builder, S.E., eds., Protein Purification: From Molecular Mechanisms to Large-Scale Processes. ACS Symposium Series No. 427, American Chemical Society, pp. 181-193 (1990)), die bevorzugt nach einem Erkennungsmotiv aus ≥ 2 Aminosäuren spalten. Dazu gehören z.B. die Prohormon-Konvertasen Furin, PC1/PC3, PC2, PC4, PC5 und PACE4 (Perona, J.J. und Craik, C.S., Protein Science 4 (1995) 337-360), die Blutgerinnungsproteasen FVIIa, FIXa, FXa, Thrombin, Protein C, Kallekrein, Plasmin, Plasminogen Aktivator und Urokinase (Furie, B. und Furie, B.C., Cell 53 (1988) 505- 518; Davie, E.W. et al., Biochem. 30 (1991) 10363-10370; WO 97/03027) und einige Proteasen viralen, wie z.B. die TEV Protease (Parks, T.D. et al., Anal. Biochem. 216 (1994) 413-417), und/oder Proteasen mikrobiellen Ursprungs, wie z.B. die Kexin (Kex2p) Protease aus Hefe (EP-A 0 467 839) und die IgA Protease aus Neisseria gonorrhoeae (Pohlner, J. et al., Nature 325 (1987) 458-462).

Zur rekombinanten Herstellung von Peptidhormonen und einigen therapeutisch genutzten Proteinen aus Fusionsproteinen werden spezifisch spaltende Proteasen, wie z.B. Trypsin, Carboxypeptidase B, Thrombin, Faktor Xa, Collagenase und Enterokinase, als Einsatzstoffe benötigt. Die Methodik der enzymatischen Spaltung von Fusionsproteinen ist Stand der Technik und die zur Spaltung von Fusionsproteinen kommerziell zur Verfügung stehenden Proteasen sind beschrieben (Flaschel, E. und Friehs, K., Biotech. Adv. 11 (1993) 31-78; Sassenfeld, H.M., Trends Biotechnol. 8 (1990) 88-93). In der Regel werden die dort aufgeführten Proteasen aus tierischen Rohstoffen, wie z.B. Pankreas, Leber und Blut, isoliert. Die aus tierischen Rohstoffen isolierten Einsatzstoffe sind jedoch nicht ideal, da sie mit infektiösen Agenzien, wie z.B. humanpathogenen Viren und Prionen, kontaminiert sein können. Trotz entsprechender Maßnahmen bei der Präparation von Proteinen / Enzymen aus tierischen und/oder menschlichen Rohstoffen (z.B. Blut) bleibt ein Restrisiko hinsichtlich der Infektion / Entstehung lebensbedrohlicher Krankheiten, wie z.B. AIDS, Hepatitis und spongiforme Enzephalopathien (Creutzfeld-Jakob-ähnlichen Krankheiten, Stichwort: BSE), bestehen. Da eine Vielzahl von Proteasen, zu denen unter anderen Trypsin, Thrombin und Faktor X zählen, in Form einer inaktiven (zymogenen) Vorstufe synthetisiert werden, wird selbst bei der rekombinanten Herstellung dieser Proteasen in der Regel noch eine zweite, aus einer tierischen Rohstoffquelle stammende Protease, zur enzymatischen Aktivierung der rekombinanten Protease benötigt. So wird z.B. zur Aktivierung von Trypsinogen Enterokinase (Hedstrom, L. et al., Science 255 (1992) 1249-1253), zur Aktivierung von Faktor X (Takeya, H. et al., J. Biol. Chem. 267 (1992) 14109-14117; WO 97/03027) und Thrombin (DiBella, E.E. et al., J. Biol. Chem. 270 (1995) 163-169) eine Protease aus Schlangengift eingesetzt.

Zudem spielen spezifisch spaltende Proteasen in der Medizin eine bedeutende Rolle. So werden z.B. FIX, FVII, tPA ("tissue type plasminogen activator"), Protein C und Thrombin und/oder davon abgeleitete Proteasevarianten bei der Behandlung von Gerinnungsstörungen eingesetzt bzw. befinden sich in der Erprobung. Außerdem ist Trypsin Bestandteil einiger Arzneimittel (Salben, Dragees und zu inhalierenden Aerosolen (Rote Liste, 1997; The United States Pharmacopeia, The National Formulary, USP23-NF18, 1995).

Von Graf, E. et al. (Biochem. 26 (1987) 2616-2623; Proc. Natl. Acad. Sci. USA 85 (1988) 4961-4965) wurde die Expression / Sekretion von Ratten Trypsinogen und Trypsinogenmutanten in E. coli beschrieben. Zur Sekretion der Trypsinogene ins Periplasma von E. coli wurde die native Trypsinogen Signalsequenz durch die Signalsequenz der bakteriellen alkalischen Phosphatase (phoA) ausgetauscht. Die sekretierten inaktiven Trypsinogene wurden aus dem Periplasma isoliert und durch enzymatische Spaltung mittels gereinigter Enterokinase aktiviert.

Von Vasquez, J.R. et al. (J. Cell. Biochem. 39 (1989) 265-276) wurde die Expression / Sekretion von anionischem Ratten Trypsin und Trypsinmutanten in E. coli beschrieben. Zur Expression / Sekretion der aktiven Trypsine ins Periplasma von E. coli wurde das native Trypsinogen prepro-Segment (Signalsequenz und Aktivierungspeptid) durch die Signalsequenz der bakteriellen alkalischen Phosphatase (phoA) ersetzt und der durch Phosphat regulierbare phoA Promotor verwendet. Aktives Trypsin wurde aus dem Periplasma isoliert. Die Ausbeute war jedoch sehr gering (ca. 1 mg/l).

Von Higaki, J.N. et al. (Biochem. 28 (1989) 9256-9263) wurde die Expression / Sekretion von Trypsin und Trypsinmutanten ins Periplasma von E. coli unter Verwendung des tac Promotors und der S. typhimurium hisJ Signalsequenz beschrieben. Die Ausbeute an aktivem Trypsin betrug ca. 0,3 mg/l. Durch Hochzelldichtefermentation konnte die Volumenausbeute von aktivem anionischem Rattentrypsin auf ca. 50 mg/l gesteigert werden (Yee, L. und Blanch, H.W., Biotechnol. Bioeng. 41 (1993) 781-790). Die Autoren weisen auf einige Probleme bei der Expression / Sekretion von aktivem Trypsin in E. coli hin. Enzymatisch aktives Trypsin entsteht im Periplasma von E. coli nach Abspaltung der Signalsequenz und nativer Trypsinproteinfaltung unter Ausbildung von 6 Disulfidbrücken. Die Bildung von aktivem Trypsin ist für die Zelle toxisch. Aktives Trypsin hydrolysiert die periplasmatischen E.coli Proteine, wodurch die Zellen lysieren. Zudem scheint die Proteinfaltung von Trypsin, im Speziellen die korrekte native Ausbildung der 6 Disulfidbrücken im Periplasma von E. coli, erschwert zu sein. Das System war nicht geeignet zur Isolierung größerer Mengen Trypsin (> 10 mg; Willet, W.S. et al., Biochem. 34 (1995) 2172-2180).

Zur Herstellung größerer Mengen Trypsin (50-100 mg) für röntgenkristallographische Untersuchungen wurde eine inaktive Trypsinogen Vorstufe in Hefe unter Kontrolle eines regulierbaren ADH/GAPDH Promotors und durch Fusion mit der Hefe α-Factor Leadersequenz sezerniert. Das ins Medium sezernierte Trypsinogen Zymogen wurde in vitro mittels Enterokinase in Trypsin überführt. Die Ausbeute betrug 10-15 mg/l (Hedstrom, L. et al., Science 255 (1992) 1249-1253).

In der EP-A 0 597 681 werden DNA Sequenzen beschrieben, die für reifes Trypsin bzw. Trypsinogen mit vorangehendem Methionrest kodieren.

In der WO 97/00316 wird ein Verfahren zur Herstellung von Trypsin oder eines Derivates durch ein rekombinantes Verfahren in Aspergillus beschrieben. Zur Transformation wird ein Vektor verwendet, welcher für Trypsinogen oder ein Derivat davon codiert, welches N-terminal an ein Signalpeptid fusioniert ist.

Aufgabe der Erfindung ist es, ein einfaches Verfahren zur rekombinanten Herstellung von Proteasen sowei neue autokatalytisch aktivierbare Proteasen sowie deren Zymogene (inaktive, zymogene Vorstufen) zur Verfügung zu stellen.

Gegenstand der Erfindung ist ein Verfahren zur rekombinanten Herstellung einer Serinprotease, gekennzeichnet durch:
a) Transformation einer prokaryontischen Wirtszelle mit einer rekombinanten Nukleinsäure, welche für eine zymogene Vorstufe der genannten Serinprotease codiert, wobei in der zymogenen Vorstufe eine natürlich vorkommende nicht-autokatalytische Spaltstelle durch eine autokatalytische Spaltstelle ersetzt ist, die von der aktiven Form der Protease erkannt wird und dabei die zymogene Vorstufe in die aktive Form spaltet,
b) Kultivierung der Wirtszelle und Expression der Nukleinsäure,
c) Isolierung der im Cytoplasma entstandenen und die zymogene Vorstufe enthaltenden inclusion bodies,
d) Naturierung der zymogenen Vorstufe und
e) autokatalytische Spaltung der zymogenen Vorstufe in die aktive Form der Serinprotease.

Es hat sich überraschenderweise gezeigt, dass mit dem erfindungsgemäßen Verfahren aktive Serinproteasen einfach und in hoher Ausbeute herstellbar sind.

Unter Serinproteasen im Sinne der Erfindung sind sowohl eukaryonische als auch mikrobielle Proteasen zu verstehen. Beispiele hierfür sind in der Einleitung zur Beschreibung dieser Erfindung genannt. Besonders vorteilhaft ist dieses Verfahren zur rekombinanten Herstellung von Trypsin, Thrombin und Faktor Xa..

Ein weiterer Gegenstand der Erfindung ist eine autokatalytisch spaltbare (aktivierbare) zymogene Vorstufe einer Serinprotease, wobei in der zymogenen Vorstufe eine natürlich vorkommende nicht-autokatalytische Spaltstelle durch eine autokatalytische Spaltstelle ersetzt ist (nicht autokatalytisch aktivierbar ist).

Als prokaryontische Wirtszelle im Sinne der Erfindung ist jede Wirtszelle zu verstehen, in der Proteine als "inclusion bodies" entstehen können. Vorzugsweise handelt es sich bei prokaryontische Wirtszellen, um E.coli-Zellen. Solche Verfahren sind beispielsweise beschrieben in F.A.O. Marston, Biochem. J. 240 (1986) 1-12: L. Stryer, Biochemistry, W.H. Freeman and Company, San Francisco, 1975, 24-30; T.E. Creighton, Progress Biophys. Molec. Biol. 33 (1978) 231-291; C.H. Schein, Bio/Technology 8 (1990) 308-317; EP-B 0 114 506; A. Mitraki und J. King, Bio/Technology 7 (1989) 690-697; EP-B 0 219 874; EP-B 0 393 725 und EP-B 0 241 022. Danach ist unter "inclusion bodies" im wesentlichen unlösliches, denaturiertes und inaktives Protein zu verstehen, welches im Cytoplasma der Wirtszellen angesammelt wird und zumindest teilweise mikroskopisch sichtbare Partikel darstellt.

Ein weiterer Gegenstand der Erfindung ist demzufolge eine Präparation einer erfindungsgemäßen autokatalytisch spaltbaren zymogenen Vorstufe einer Serinprotease, dadurch gekennzeichnet, dass das zu präparierende Protein inaktiv und denaturiert in Form von inclusion bodies in einer prokaryontischen Zelle vorliegt. Ein weiterer Gegenstand der Erfindung ist eine wässrige Lösung, die aus einem Denaturierungsmittel in einer Konzentration, die geeignet ist, die inclusion bodies aufzulösen, und den aufgelösten inclusion bodies besteht.

Unter einer zymogenen (inaktiven) Vorstufe einer Serinprotease (Zymogen) ist ein Protein zu verstehen, welches auch nach Naturierung der inclusion bodies keine oder nur eine sehr geringe proteolytische

Aktivität, im Gegensatz zur aktiven Protease in der korrekten Proteinstruktur, zeigt (mindestens um den Faktor 5, vorzugsweise den Faktor 10 geringere Aktivität als die aktive Form). Die zymogene Form unterscheidet sich von der aktiven Form der Protease im wesentlichen darin, dass sie zusätzliche Aminosäuren enthält, an welchen gespalten oder die abgespalten werden müssen, um die aktive Form der Protease zu erhalten. Diese Aminosäuren könne C-terminal, N-terminal und/oder innerhalb der Protease lokalisiert sein.

Proteasen wie Faktor IX, Faktor X, Thrombin oder Plasminogenaktivator bestehen aus mehreren Domänen. Eine dieser Domänen ist die Proteasedomäne, also die Domäne, welche die Proteaseaktivität vermittelt. Eine Vorstufe der Protease ist im erfindungsgemäßen Sinne dann inaktiv, wenn am N-Terminus der Proteasedomäne zusätzliche Aminosäuren enthalten sind, die autokatalytisch abspaltbar sind.

Unter Naturierung im Sinne der Erfindung ist ein Verfahren zu verstehen, bei welchem ein denaturiertes und im wesentlichen inatkives Protein in eine Konformation übergeführt wird, in der das Protein nach autokatalytischer Spaltung die gewünschte Aktivität zeigt. Üblicherweise handelt es sich bei dieser Konformation um die natürlicherweise vorhandene Konformation des Proteins. Zur Naturierung wird durch dem Fachmann geläufige Verfahren (siehe oben) das schwer lösliche denaturierte Protein in Denaturierungsmitteln, wie Guanidinhydrochlorid oder Harnstoff, gelöst, gegebenenfalls reduziert und durch Verringerung der Konzentration des Denaturierungsmittels und/oder gegebenenfalls durch Zugabe einer Denaturierungshilfe, wie Arginin, und/oder eines Redoxsystems, wie GSH/GSSG, in seine aktive Konformation übergeführt.

Unter einer autokatalytischen Spaltung im Sinne der Erfindung ist eine Spaltung der zymogenen Vorstufe der Serinprotease in die aktive Form zu verstehen, welche ohne Zusatz von weiteren Enzymen erfolgt. Da die zymogenen Vorstufen der Proteasen üblicherweise noch geringe restliche proteolytische Aktivitäten zeigen, ist dies für den Start der autokatalytischen Proteolyse ausreichend. Vorzugsweise beträgt das Verhältnis der proteolytischen Aktivität der zymogenen Vorstufe und der aktiven Protease 1 : 5 oder weniger.

Die rekombinante Herstellung von Proteasen ist schwierig zu bewerkstelligen, da diese Proteasen als aktives Protein die Proteine des Wirtsorganismus spalten und dadurch letal für den Wirtsorganismus sind. Wenn die Proteasen in inaktiver, zymogener Form rekombinant hergestellt werden, ist es erforderlich, das Protein nach der rekombinanten Herstellung in einem zusätzlichen Schritt in die aktive Form zu überführen. Zur Spaltung der zymogenen Form werden wiederum Proteasen, die üblicherweise aus natürlichen Quellen, wie tierischen Rohstoffen, gewonnen werden, verwendet. Damit ist ein solches Verfahren aufwendig und kostenintensiv.

Das erfindungsgemäße Verfahren zeichnet sich dagegen dadurch aus, dass auf einen zusätzlichen Schritt zur Spaltung der zymogenen Form, durch Zusatz einer weiteren Protease, vollständig verzichtet werden kann. Außer der Kostenersparnis, hat dies auch den Vorteil, dass die so hergestellte rekombinante Serinprotease nicht durch weitere Proteasen (insbesondere durch Proteasen tierischer Herkunft) oder durch Proteine, die natürlicherweise als Verunreinigung der Proteasen bei der Isolierung aus natürlichen Quellen (z.B. Säugerzellenproteine) gefunden werden, verunreinigt ist.

Die inaktive, zymogene Form der Serinprotease kann bei der rekombinanten Herstellung, wie jedes andere Protein, exprimiert, isoliert und aufgereinigt werden. Solche Verfahren sind dem Fachmann sowohl für eukaryontische als auch prokaryontische Zellen bekannt.

Proteasen, die erfindungsgemäß hergestellt werden können, sind spezifisch spaltende Proteasen, wie insbesondere Serinproteasen (z.B. die eukaryontischen Proteasen, wie Faktor IX, Faktor X, Faktor VII, Protein C, Thrombin, Trypsin, Chymotrypsin oder Gewebeplasminogenaktivator).

Erkennungssequenz(en) und Positionierung der in die Protease eingefügten autokatalytischen Spaltstelle oder Spaltstellen sind vom Typ der Protease und auch davon abhängig, wie die Aktivierung der zymogenen Form erfolgt. Die aktive Form von Faktor X entsteht beispielsweise dadurch, dass aus der inaktiven, zymogenen Form ein Fragment herausgeschnitten wird. Dies bedeutet, dass erfindungsgemäß am N- und C-Terminus dieses Fragment jeweils eine autokatalytische Spaltstelle vorhanden sein muss. Die Aktivierung von Trypsin verläuft in der Weise, dass am N- Terminus der zymogenen Form ein Aktivierungspeptid abgespalten wird. In diesem Fall ist es also erforderlich, das native Aktivierungspeptid so zu verändern, dass es autokatalytisch gespalten werden kann. Bei Plasminogenaktivatoren, wie Gewebsplasminogenaktitvator, erfolgt die Aktivierung nur durch Spaltung der Peptidbindung innerhalb des Moleküls. Erfindungsgemäß wird in einem solchen Falle also die natürlicherweise vorkommende Spaltstelle durch die autokatalytische Spaltstelle ersetzt.

Bei der Endoproteinase LysC erfolgt die Aktivierung durch Abspaltung von jeweils einem N- und C-terminalen Fragment aus einer prepro-Form während der Sekretion. In diesem Fall sollte erfindungsgemäß das C-terminale Prosegment entfernt werden und zwischen das N-terminale Prosegment und die Proteasedomäne eine geeignete autokatalytische Spaltstelle eingefügt werden.

In einer bevorzugten Ausführungsform der Erfindung unterscheidet sich die rekombinante zymogene Form der Serinprotease von der natürlicherweise vorkommenden zymogenen Form. Beispielsweise enthalten bakterielle Proteasen in der zymogenen Form (zelluläre Vorläuferform) Signalsequenzen und Prosequenzen, die dazu geeignet sind, die Ausschleusung der inaktiven Protease aus der Zelle zu ermöglichen. Beim erfindungsgemäßen Verfahren entsteht die rekombinante Protease in Form von unlöslichen "inclusion bodies". Es ist also vorteilhaft, die Aminosäuresequenz der zymogenen Form so zu optimieren, dass sie möglichst vollständig zur Bildung von "inclusion bodies" führt, aus denen die Protease zweckmäßig mit hoher Ausbeute naturierbar ist. Da die "inclusion bodies" aufgrund der denaturierten Form des Proteins keine enzymatische Aktivität zeigen, ist es nicht unbedingt erforderlich, dass die zymogene Form überhaupt keine proteolytische Aktivität zeigt. Es ist sogar bevorzugt, dass die zymogene Form eine gringe proteolytische Aktivität zeigt, um die autokatalytische Spaltung nach Naturierung zu beschleunigen.

Vorzugsweise erfolgt die Einfügung der Spaltstelle oder der Spaltstellung unter Beibehaltung der nativen Primärstruktur (Proteasedomäne). Erfindungsgemäß ist es dadurch beispielsweise möglich, aktives Trypsin mit unveränderter Aminosäuresequenz auf einfache Weise in Prokaryonten herzustellen. Damit wird eine Protease erhalten, bei der nicht mehr das N-terminale Startcodon (Methionin) abgespalten werden muss.

In einer weiteren bevorzugten Ausführungsform wird die erfindungsgemäß hergestellte aktive Serinprotease immobilisiert. Eine solche Immobilisierung kann beispielsweise durch Bindung an einen polymeren, unlöslichen Träger erfolgen oder durch Selbstvemetzung (vgl. z.B. US-Patent 4,634,671 und EP-A 0 367 302).

Die zymogene Form kann im wesentlichen (bis auf die autokatalytische Spaltstelle) der natürlichen zymogenen Form der Protease entsprechen. Im Falle von Faktor X enthält die natürlicherweise vorkommende zymogene Form bereits eine autokatalytische Spaltstelle. In diesem Fall ist die zweite Spaltstelle der zymogenen Form durch eine weitere autokatalytische Spaltstelle ersetzt. In einer bevorzugten Ausführungsform ist die Sequenz des abzuspaltenden Fragments oder der abzuspaltenden Fragmente weiter modifiziert. Geeignete autokatalytische Spaltstellen sind für bevorzugte Enzyme in nachfolgender Tabelle angegeben:

### Restriktionsendoprotease / Spaltstelle

- Enterokinase (Asp)₄Lys↓
- Faktor Xa (Rind) IleGluGlyArg↓
- Faktor Xa (Mensch) IleAspGlyArg↓
- Thrombin ArgGlyProArg↓
- TEV Protease GluAsnLeuTyrPheGln↓Gly/Ser
- IgA Protease YyyPro↓XxxPro, Yyy = Pro, Ala, Gly, Thr; Xxx = Thr, Ser, Ala
- Kex2p Protease 2 benachbarte basische Aminosäuren (Lys oder Arg)
- LysC Endoprotease Lys↓
- Trypsin Lys↓ oder Arg↓
- Clostripain Arg↓
- S. aureus V8 Glu↓

Eine bevorzugte Ausführungsform der Erfindung ist eine autokatalytisch aktivierbare zymogene Vorstufe von Faktor X. Faktor X ist eine komplexe aus mehreren Domänen bestehende glycosylierte Protease. Er gehört mechanistisch zur Familie der Serinproteasen. FX wird in der Leber als inaktives Proenzym (Zymogen) synthetisiert, ins Blut sezerniert und bei Bedarf durch spezifische Proteolyse aktiviert. Der Faktor X ist hinsichtlich der Proteindomänenanordnung analog wie der Faktor VII, IX und Protein C aufgebaut. Zudem sind die Aminosäuresequenzen dieser 4 Proteasen sehr homolog (Aminosäuresequenz-Identität: ca. 40%). Sie werden zu einer Proteaseunterfamilie, der Faktor IX-Familie, zusammengefaßt.

Ebenfalls erfindungsgemäß bevorzugt werden die Proteasen der Faktor IX-Familie (Faktor VII, IX, X und Protein C) Diese Proteasen bestehen gemäß Furie, B. und Furie, B.C. (Cell 53 (1988) 505- 518) aus
- einem Propeptid,
- einer GLA-Domäne,
- einer "aromatic amino acid stack" Domäne,
- zwei EGF-Domänen (EGF1 und EGF2),
- einer Zymogen Aktivierungsdomäne (Aktivierungspeptid, AP) und
- einer katalytischen Proteasedomäne (CD).

Zudem werden die Blutplasmaproteasen während der Sekretion posttranslational modifiziert:
- 11 - 12 Disulfidbrücken
- N- und/oder O-Glycosylierung (GLA-Domäne und Aktivierungspeptid)
- Bharadwaj, D. et al., J. Biol. Chem. 270 (1995) 6537-6542
- Medved, L.V. et al., J. Biol. Chem. 270 (1995) 13652-13659
- Abspaltung des Propeptides
- γ-Carboxylierung von Glu-Resten (GLA-Domäne)
- β-Hydroxylierung eines Asp-Restes (EGF-Domänen)

Nach Aktivierung der Zymogene (zymogene Form des Proteins) durch spezifische Spaltung von ein oder zwei Peptidbindungen (Abspaltung eines Aktivierungspeptids) bestehen die enzymatisch aktiven Proteasen aus 2 Ketten, die entspechend ihrem Molekulargewicht mit schwerer und leichter Kette bezeichnet werden. Die beiden Ketten werden in der Faktor IX-Proteasefamilie durch eine intermolekulare Disulfidbrücke zwischen der EGF2-Domäne und der Proteasedomäne zusammengehalten. Die Zymogen-Enzym Transformation (Aktivierung) führt zu Konformationsänderungen innerhalb der Proteasedomäne. Dadurch kann sich eine für die Proteaseaktivität notwendige essentielle Salzbrücke zwischen der α-NH₃⁺-Gruppe der N-terminalen Aminosäure der Proteasedomäne und einem Asp-Rest innerhalb der FXa-Proteasedomäne ausbilden. Für diese Untergruppe von Serinproteasen ist der N-terminale Bereich sehr kritisch und darf nicht modifiziert werden. Nur dann kann sich die für Serinproteasen typische "active site" mit der katalytischen Triade aus Ser, Asp und His ausbilden (Blow, D.M.: Acc. Chem. Res. 9 (1976) 145-152; Polgar, L.: In: Mechanisms of protease action. Boca Raton, Florida, CRC Press, chapter 3 (1989)).

Die FX Aktivierungspeptid-Prozessierung beginnt bereits in der Zelle während der Sekretion (erste Spaltung zwischen der EGF2-Domäne und dem Aktivierungspeptid). Durch eine zweite FIXa- oder FVIIa-katalysierte Spaltung an der Membran im Komplex mit dem Cofaktor FVIIIa bzw. "tissue factor" wird dann FX zu FXa aktiviert (Mann, K. G. et al., Blood 76 (1990) 1-16).

Die katalytische Domäne von FXa besteht aus 254 Aminosäuren, ist nicht glycosyliert und bildet 4 Disulfidbrücken aus. Sie ist strukturell aus 2 "tonnenförmigen" β-Faltblättem aufgebaut, den sogenannten Halbseiten.

Die Herstellung von verkürzten posttranslational nicht modifizierten Blutplasmaproteasevarianten der Faktor IX-Familie (Faktor VII, IX, X und Protein C) bestehend aus EGF2-Domäne, Aktivierungspeptid (AP) und katalytischer Domäne (CD) durch Expression der entsprechenden Gene in E. coli und anschließende Naturierung und Aktivierung der inaktiven Proteaseproteine in vitro ist in der WO 97/03027 umfassend beschrieben.

Ebenfalls erfindungsgemäß bevorzugt sind autokatalytisch spaltbare zymogene Vorstufen von Trypsinproteasen. Die Trypsinproteasen werden in den exokrinen Acinuszellen des Pankreas als inaktive Proenzyme (Zymogene) den sogenannten Trypsinogenen gebildet. Aus dem menschlichen Pankreassekret wurden 4 unterschiedlicheTrypsinogene (Trypsinogen I, II, III und IV) isoliert, enzymatisch charakterisiert und die Aminosäuresequenzen ermittelt. Die 2 am stärksten exprimierten Trypsinogen Gene TRYI (Trypsinogen I) und TRYII (Trypsinogen II) sind bekannt. Sie wurden durch Klonierung der entsprechenden cDNAs (Emi, M. et al., Gene 41 (1986) 305-310) isoliert. Die humanen Trypsinogen Gene TRYI und TRYII kodieren gemäß einem sezernierten Protein für ein übliches Signalpeptid aus 15 Aminosäureresten. Danach folgt ein für die Trypsinogen Gene charakteristisches Prosegment, das im Falle der humanen Trypsinogene I und II aus dem N-terminalen Aktivierungspeptid AlaProPheAspAspAspAspLys besteht (Guy, O. et al., Biochem. 17 (1978) 1669-1675). Dieses Prosegment wird von der aus den Dünndarm-Mucosazellen in den Dünndarm sezernierten Glycoprotease Enterokinase erkannt und in Gegenwart von Calcium abgespalten, wodurch die inaktiven Trypsinogene in die aktive Form, die Trypsine überführt werden. Die Aktivierung der Trypsinogene verläuft teilweise autokatalytisch. Die Spaltung durch Enterokinase ist jedoch über 1000 mal schneller.

Die Trypsine gehören wie der Faktor Xa zur Familie der Serinproteasen. Die Aktivierung der Trypsinogene durch Abspaltung des N-terminalen Aktivierungspeptids führt auch hier zu einer Konformationsänderung innerhalb der Proteasedomäne unter Beteiligung des freien N-Terminus (Ausbildung einer essentiellen Salzbrücke zwischen der α-NH₃⁺-Gruppe der N-terminalen Aminosäure von Trypsin und dem Asp194-Rest innerhalb der Proteasedomäne), wodurch sich die für Serinproteasen typische "active site" mit der katalytischen Triade aus Ser, Asp und His ausbilden kann.

Das humane am stärksten exprimierte Trypsinogen I Gen (TRYI) kodiert für 247 Aminosäuren inklusive einer Signalsequenz aus 15 Aminosäuren und einem Aktivierungpeptid aus 8 Aminosäuren. Das reife Trypsin I Isoenzym besteht somit aus 224 Aminosäureresten. Es enthält 10 Cysteinreste, die 5 Disulfidbrücken ausbilden (Emi, M. et al., Gene 41 (1986) 305-310). Die katalytische Domäne von Trypsin ist analog wie FXa strukturell aus 2 "tonnenförmigen" β-Faltblättern aufgebaut.

Das humane Trypsinisoenzym I besitzt zu dem humanen Trypsinisoenzym II eine Sequenzhomologie von 89%, zu Rindertrypsin eine Sequenzhomologie von ca. 75% und zu der katalytischen Domäne des humanen Faktors Xa eine Sequenzhomologie von ca. 43%.

Die folgenden Beispiele, Publikationen, das Sequenzprotokoll und die Figuren erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

Im Sequenzprotokoll bedeuten:
- **SEQ ID NO:1 - SEQ ID NO:10**: Primer N1 - N10.
- **SEQ ID NO: 11**: das native FX Aktivierungspeptid.
- **SEQ ID NO:12**: die Nukleotidsequenz des klonierten TRYI-Variantengens.

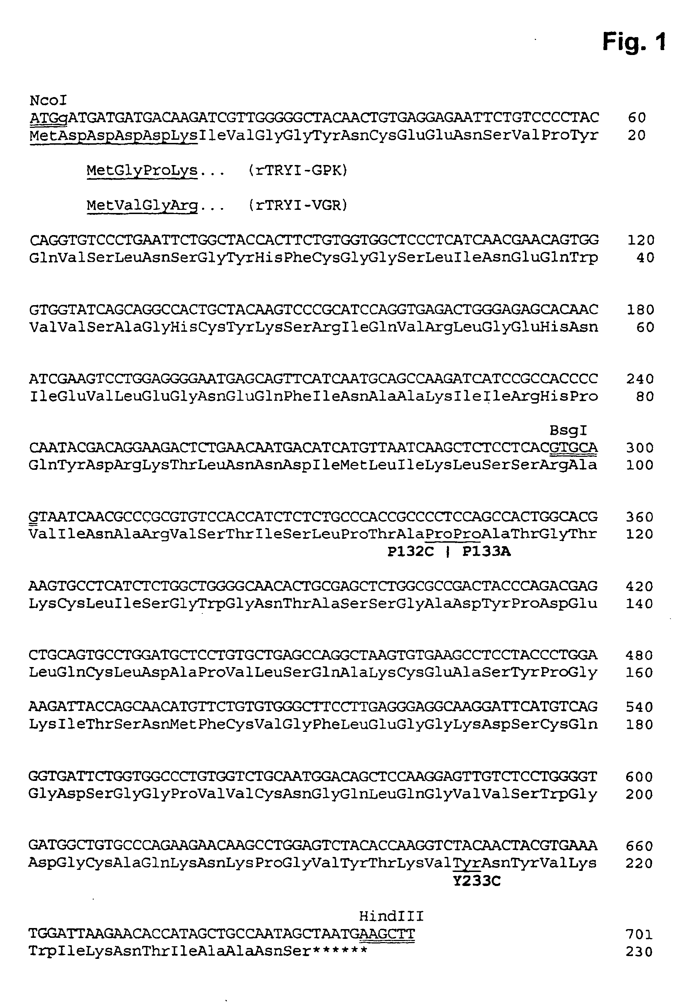

- **Fig. 1**: zeigt die Nukleotid- und abgeleitete Aminosäuresequenz des klonierten TRYI Trypsin-Variantengens mit verkürztem Prosegment. Die modifizierten Aktivierungspeptide der autokatalytisch aktivierbaren Trypsinvarianten rTRYI-GPK und rTRYI-VGR sind eingezeichnet. Die in der TRYI-GPK-SS Variante zusätzlich eingeführten Mutationen (P132C, P133A und Y233C; Aminosäuresequenz-Nummerierung entsprechend der Publikation von Emi, M. et al. (Gene 41 (1986) 305-310) sind hervorgehoben.

### Beispiele

### Methoden

### Rekombinante DNA-Technik

Zur Manipulation von DNA wurden Standardmethoden benutzt, wie sie bei Sambrook, J. et al. (1989) In: Molecular cloning: A laboratory manual. Cold Spring Harbor Laboratory Press, Cold Spring Harbor, New York, beschrieben sind. Die verwendeten molekularbiologischen Reagenzien wurden nach den Angaben des Herstellers eingesetzt.

### Proteinbestimmung

Die Proteinkonzentration der Proteasen und Proteasevarianten wurde durch Bestimmung der optischen Dichte (OD) bei 280 nm unter Verwendung des anhand der Aminosäuresequenz errechneten molaren Extinktionskoeffizienten ermittelt.

### Expressionsvektor

Der Vektor zur Expression der autokatalytisch aktivierbaren Proteasen basiert auf dem Expressionsvektor pSAM-CORE für core-Streptavidin. Die Herstellung und Beschreibung des Plasmids pSAM-CORE ist in der WO 93/09144 von Kopetzki, E. et al. beschrieben. Das core-Streptavidin Gen wurde durch das gewünschte Proteasegen im pSAM-CORE Vektor ersetzt.

### Beispiel 1

### Klonierung des humanen Trypsinogen I Gens (Plasmid: pTRYI)

Die Trypsinogen I cDNA von Bp-Position 61-750, kodierend für Trypsinogen I von Aminosäureposition 19-247 (cDNA Sequenz- und Aminosäuresequenz-Nummerierung entsprechend der Publikation von Emi, M. et al. (Gene 41 (1986) 305-310) wurde in einer "Polymerase Chain Reaktion" (PCR) gemäß der Methode von Mullis, K.B. und Faloona, F.A. (Methods Enzymol. 155 (1987) 355-350) unter Verwendung der PCR Primer N1 (SEQ ID NO: 1) und N2 (SEQ ID NO: 2) und einer kommerziell erhältlichen humanen Leber cDNA Genbank (Vektor: Lambda ZAP® II) der Firma Stratagene (La Jolla, CA, U.S.A.) als Template DNA amplifiziert. Mittels der PCR Primer wurde am 5'-Ende der kodierenden Region eine singuläre NcoI Schnittstelle und ein ATG-Startkodon und am 3'-Ende der kodierenden Region eine singuläre HindIII Schnittstelle eingeführt (Fig. 1).

Das ca. 715 Bp lange PCR Produkt wurde mit den Restriktionsendonukleasen NcoI und HindIII verdaut und das ca. 700 Bp lange NcoI/HindIII-Trypsinogen I Fragment nach Reinigung durch Agarosegelelektrophorese in das ca. 2,55 kBp lange NcoI/HindIII-pSAM-CORE Vektorfragment ligiert. Das gewünschte Plasmid pTRYI wurde durch Restriktionskartierung identifiziert und die durch PCR isolierte TRYI cDNA Sequenz durch DNA Sequenzierung überprüft.

### Beispiel 2

### Konstruktion des Trypsinvariantengens TRYI-GPK (autokatalytisch aktivierbare Trypsinvariante; Plasmid: pTRYI-GPK)

Das N-terminale native Trypsinogen I Prosegment AlaProPheAspAspAspAspLys (Guy, O. et al., Biochem. 17 (1978) 1669-1675) wurde durch das Aktivierungspeptid GlyProLys ersetzt. Das gewünschte TRYI-GPK Trypsinvariantengen wurde mittels PCR-Technik hergestellt.

Dazu wurde die humane Trypsinogen I cDNA von Bp-Position 73-750, kodierend für Lys-Trypsin I von Aminosäureposition 23-247 (cDNA Sequenz- und Aminosäuresequenz-Numerierung entsprechend der Publikation von Emi, M. et al. (Gene 41 (1986) 305-310) mittels PCR unter Verwendung der Primer N3 (SEQ ID NO: 3) und N2 (SEQ ID NO: 2, Beispiel 1) und dem Plasmid pTRYI (Beispiel 1) als Template DNA amplifiziert. Mittels des 5'-überhängenden Endes des PCR Primers N3 wurde eine DNA Sequenz kodierend für ein ATG-Startkodon und das Aktivierungspeptid GlyProLys mit einer singulären NcoI Schnittstelle am 5'-Ende eingeführt. Zudem wurde mit dem PCR Primer N3 innerhalb der kodierenden N-terminalen Region des Trypsin I Strukturgens eine zusätzliche MunI Schnittstelle eingeführt und teilweise die "Codonusage" (Aminosäurepositionen: 7, 8 und 10) an die in E. coli bevorzugt benutzten Kodons angepaßt ("ATG-Umgebung mit optimierter Codonusage", gekennzeichnet durch Kleinschreibung der Basen im N3 Primer), ohne die Proteinsequenz zu verändern.

Das ca. 700 Bp lange PCR Produkt wurde mit den Restriktionsendonukleasen NcoI und HindIII verdaut und das ca. 685 Bp lange NcoI/HindIII-TRYI-GPK Fragment nach Reinigung durch Agarosegelelektrophorese in das ca. 2,55 kBp lange NcoI/HindIII-pSAM-CORE Vektorfragment ligiert. Das gewünschte Plasmid pTRYI-GPK wurde durch Restriktionskartierung identifiziert (zusätzliche MunI Schnittstelle) und das durch PCR amplifzierte TRPI-GPK Gen durch DNA Sequenzierung überprüft.

### Beispiel 3

### Konstruktion des Trypsinvariantengens TRYI-VGR (autokatalytisch aktivierbare Trypsinvariante; Plasmid: pTRYI-VGR)

Das N-terminale native Trypsinogen I Prosegment AlaProPheAspAspAspAspLys wurde durch das Aktivierungspeptid ValGlyArg ersetzt. Das TRYI-VGR Trypsinvariantengen wurde analog wie das TRYI-GPK Trypsinvariantengen mittels PCR-Technik hergestellt.

Dazu wurde die humaneTrypsinogen I cDNA von Bp-Position 76-750, kodierend für Trypsin I von Aminosäureposition 24-247 (cDNA Sequenz- und Aminosäuresequenz-Nummerierung entsprechend der Publikation von Emi, M. et al. (Gene 41 (1986) 305-310) mittels PCR unter Verwendung der Primer N4 (SEQ ID NO: 4) und N2 (SEQ ID NO: 2, Beispiel 1) und dem Plasmid pTRYI (Beispiel 1) als Template DNA amplifiziert. Mittels des 5'-überhängenden Endes des PCR Primers N4 wurde eine DNA Sequenz kodierend für ein ATG-Startkodon und das Aktivierungspeptid ValGlyArg mit einer singulären NcoI-Schnittstelle am 5'-Ende eingeführt. Zudem wurde mit dem PCR Primer N4 innerhalb der kodierenden N-terminalen Region des Trypsin I Strukturgens eine zusätzliche MunI Schnittstelle eingeführt und teilweise die "Codonusage" (Aminosäurepositionen: 7, 8 und 10) an die in E. coli bevorzugt benutzten Kodons angepaßt ("ATG-Umgebung mit optimierter Codonusage", gekennzeichnet durch Kleinschreibung der Basen im N4 Primer), ohne die Proteinsequenz zu verändern.

Das ca. 700 Bp lange PCR Produkt wurde mit den Restriktionsendonukleasen NcoI und HindIII verdaut und das ca. 685 Bp lange NcoI/HindIII-TRYI-VGR Fragment nach Reinigung durch Agarosegelelektrophorese in das ca. 2,55 kBp lange NcoI/HindIII-pSAM-CORE Vektorfragment ligiert. Die Herstellung und Beschreibung des Plasmids pSAM-CORE ist in der WO 93/09144 von Kopetzki, E. et al. beschrieben. Das gewünschte Plasmid pTRYI-VGR wurde durch Restriktionskartierung identifiziert (zusätzliche MunI Schnittstelle) und das durch PCR amplifizierte TRPI-VGR Gen durch DNA Sequenzierung überprüft.

### Beispiel 4

### Konstruktion des Trypsinvariantengens TRYI-GPK-SS (autokatalytisch aktivierbare Trypsinvariante mit einer zusätzlichen Disulfidbrücke; Plasmid: pTRYI-GPK-SS)

Durch Einführung einer zusätzlichen Disulfidbrücke wurde das humane Trypsin I Isoenzym verändert. Dazu wurde die Aminosäure Pro an Position 132 und die Aminosäure Tyr an Position 233 zu Cys mutiert (Aminosäuresequenz-Nummerierung entsprechend der Publikation von Emi, M. et al. (Gene 41 (1986) 305-310)). Durch diese 2 Punktmutationen (P**132**C und Y**233**C) kann sich eine zusätzliche 6-te Disulfidbrücke zwischen den erfindungsgemäß eingeführten Cysteinresten an Position 132 und 233 ausbilden. Zudem wurde die Aminosäure Pro an der Position 133 zu Ala mutiert (P**133**A).

Dazu wurde die Trypsinogen I cDNA von Bp-Position 353-750, kodierend für Trypsinogen I von Aminosäureposition 116-247 (cDNA Sequenz- und Aminosäuresequenz-Nummerierung entsprechend der Publikation von Emi, M. et al. (Gene 41 (1986) 305-310), mittels PCR unter Verwendung der Primer N5 (SEQ ID NO: 5) und N6 (SEQ ID NO: 6) und dem Plasmid pTRYI (Beispiel 1 ) als Template DNA amplifiziert. Mittels des PCR Primers N5 wurde die P132C und die P133A Mutation und mit dem Primer N6 die Y233C Mutation eingeführt. Die Mutationen sind durch Kleinschreibung der Basen in den PCR Primern gekennzeichnet.

Das ca. 420 Bp lange PCR Produkt wurde mit den Restriktionsendonukleasen BsgI und HindIII verdaut und das ca. 405 Bp lange BsgI/HindIII-TRYI Fragment nach Reinigung durch Agarosegelelektrophorese in das ca. 2,85 kBp lange BsgI/HindIII-pTRPI-GPK Vektorfragment ligiert (Beispiel 2). Das gewünschte Plasmid pTRYI-GPK-SS wurde durch Restriktionskartierung identifiziert (fehlende DraIII Schnittstelle) und die durch PCR amplifizierte TRYI DNA Sequenz durch DNA Sequenzierung überprüft.

### Beispiel 5

### Konstruktion des FX Proteasevariantengens FX-EGF2-APau-CD (autokatalytisch aktivierbare FX-EGF2-AP-CD Variante; Plasmid: pFX-EGF2-APau-CD)

Die Klonierung des FX Gens und die Konstruktion des Plasmids pFX-EGF2-AP-CD ist in der WO 97/03027, Beispiel 3) ausführlich beschrieben. Die FX-EGF2-AP-CD Expressionseinheit auf dem Plasmid pFX-EGF2-AP-CD kodiert für eine N-terminal verkürzte FX Proteasevariante aus EGF2-Domäne, Aktivierungspeptid und katalytischer Proteasedomäne.

Im DNA Segment kodierend für das native FX Aktivierungspeptid (AP) (SEQ-ID-NO:11) wurden die C-terminalen drei Aminosäuren Asn, Leu, Thr durch Ile, Asp, Gly ersetzt, wobei das modifizierte FX-Aktivierungspeptid APau entstand.

Die gewünschte NLT zu IDG Aminosäuresequenzänderung wurde mittels einer Fusions-PCR eingeführt.

Dazu wurde in einer ersten PCR die FX DNA von Bp-Position 330-629, kodierend für die EGF2-Domäne und das Aktivierungspeptid von Aminosäureposition 111-209 (Sequenz- und Aminosäuresequenz-Nummerierung entsprechend Fig. 3, WO 97/03027) unter Verwendung der PCR Primer N7 (SEQ ID NO: 7) und N8 (SEQ ID NO: 8) und des Plasmids pFX-EGF2-AP-CD (Herstellung und Beschreibung siehe: WO 97/03027, Beispiel 3) als Template DNA amplifiziert. Mittels des PCR Primers N8 wurde am 3'-Ende eine singuläre Van91I Schnittstelle eingeführt, ohne die Aminosäuresequenz zu verändern.

In einer zweiten PCR wurde die FX DNA von Bp-Position 619-1362 kodierend für die C-terminale Region des modifizierten FX Aktivierungspeptids und der FX Proteasedomäne von Aminosäureposition 207-454 (Sequenz- und Aminosäuresequenz-Nummerierung entsprechend Fig. 3, WO 97/03027) unter Verwendung der PCR Primer N9 (SEQ ID NO: 9) und N10 (SEQ ID NO: 10) und des Plasmids pFX-EGF2-AP-CD als Template DNA amplifiziert. Mittels des PCR Primers N9 wurde am 5'-Ende eine singuläre Van91I Schnittstelle und die gewünschte NLT zu IDG Aminosäuresequenzveränderung eingeführt.

Das ca. 300 Bp lange EGF2-AP DNA Fragment der 1-ten PCR wurde mit den Restriktionsendonukleasen PstI und Van91I und das ca. 750 Bp lange FX DNA Fragment der 2-ten PCR wurde mit den Restriktionsendonukleasen Van91I und HindIII verdaut. Nach Reinigung durch Agarosegelelektrophorese wurde das ca. 285 Bp lange PstI/Van91I-EGF2-AP DNA Fragment mit dem 740 Bp langen Van91I/HindIII-FX DNA Fragment und dem ca. 2,56 kBp PstI/HindIII pFX-EGF2-AP-CD Vektorfragment in einer Dreifragmentligation ligiert. Das gewünschte Plasmid pFX-EGF2-APau-CD wurde durch Restriktionskartierung identifiziert (zusätzliche Van91I Schnittstelle) und das FX-EGF2-APau-CD Gen durch DNA Sequenzierung überprüft.

### Beispiel 6

### a) Expression der Proteasegene in E. coli

Zur Expression der Trypsin- und FX Variantengene wurde ein E. coli K12 Stamm (z.B. UT5600; Grodberg, J. und Dunn, J.J., J. Bacteriol. 170 (1988) 1245-1253) jeweils mit einem der in den Beispielen 2-5 beschriebenen Expressionsplasmiden pTRYI-GPK, pTRYI-VGR, pTRYI-GPK-SS und pFX-EGF2-APau-CD (Ampicillin-Resistenz) und dem lacI^{q}-Repressorplasmid pUBS520 (Kanamycin-Resistenz, Herstellung und Beschreibung siehe: Brinkmann, U. et al., Gene 85 (1989) 109-114) transformiert.

Die mit den Expressionsplasmiden transformierten UT5600/pUBS520/Zellen wurden in Schüttelkultur in DYT-Medium (1% (w/v) Hefeextrakt, 1% (w/v) Bacto Tryptone, Difco, und 0,5% NaCl) mit 50-100 mg/l Ampicillin und 50 mg/l Kanamycin bei 37°C bis zu einer optischen Dichte bei 550 nm (OD₅₅₀) von 0,6-0,9 angezogen und anschließend mit IPTG (1-5 mmol/l Endkonzentration) induziert. Nach einer Induktionsphase von 4 - 8 Stunden (Std.) bei 37°C wurden die Zellen durch Zentrifugation (Sorvall RC-5B Zentrifuge, GS3 Rotor, 6000 UPM, 15 min) geerntet, mit 50 mmol/l Tris-HCl Puffer, pH 7,2 gewaschen und bis zur Weiterverarbeitung bei -20°C gelagert. Die Zellausbeute aus einer 1 1 Schüttelkultur betrug 4-5 g (Naßgewicht).

### b) Expressionsanalyse

Die Expression der mit den Expressionsplasmiden pTRYI-GPK, pTRYI-VGR, pTRYI-GPK-SS und pFX-EGF2-APau-CD transformierten UT5600/pUBS520/Zellen wurde analysiert. Dazu wurden Zellpellets aus jeweils 1 ml abzentrifugiertem Anzuchtmedium in 0,25 ml 10 mmol/l Tris-HCl, pH 7,2 resuspendiert und die Zellen durch Ultraschallbehandlung (2 Pulse a 30 s mit 50% Intensität) mit einem Sonifier® Cell Disruptor B15 der Firma Branson (Heusenstamm, Deutschland) aufgeschlossen. Die unlöslichen Zellbestandteile wurden sedimentiert (Eppendorf 5415 Zentrifuge, 14000 UPM, 5 min) und der Überstand mit 1/5 Volumen (Vol) 5xSDS-Probenpuffer (1xSDS-Probenpuffer: 50 mmol/l Tris-HCl, pH 6,8, 1% SDS, 1% Mercaptoethanol, 10% Glycerin, 0,001% Bromphenolblau) versetzt. Die unlösliche Zelltrümmerfraktion (Pellet) wurde in 0,3 ml 1xSDS-Probenpuffer mit 6-8 M Harnstoff resuspendiert, die Proben 5 min bei 95°C inkubiert und erneut zentrifugiert. Danach wurden die Proteine durch SDS-Polyacrylamid Gelelektrophorese (PAGE) aufgetrennt (Laemmli, U.K., Nature 227 (1970) 680-685) und mit Coomassie Brilliant Blue R Farbstoff angefärbt.

Die in E. coli synthetisierten Proteasevarianten waren homogen und wurden ausschließlich in der unlöslichen Zelltrümmerfraktion gefunden ("inclusion bodies", IBs). Die Expressionshöhe betrug 10-50% bezogen auf das E. coli Gesamtprotein.

### Beispiel 7

### Zell-Lyse, Solubilisierung und Naturierung der Proteasegene

### a) Zell-Lyse und Präparation der "inclusion bodies" (IBs)

Das Zellpellet aus 3 1 Schüttelkultur (ca. 15 g Naßgewicht) wurde in 75 ml 50 mmol/l Tris-HCl, pH 7,2, resuspendiert. Die Suspension wurde mit 0,25 mg/ml Lysozym versetzt und 30 min bei 0°C inkubiert. Nach Zugabe von 2 mmol/l MgCl₂ und 10 mg/ml DNase I (Boehringer Mannheim GmbH, Kat.-Nr. 104159) wurden die Zellen mechanisch mittels Hochdruckdispersion in einer French® Press der Firma SLM Amico (Urbana, IL, U.S.A.) aufgeschlossen. Anschließend wurde die DNA 30 min bei Raumtemperatur (RT) verdaut. Der Ansatz wurde mit 37,5 ml 50 mmol/l Tris-HCl pH 7,2, 60 mmol/l EDTA, 1,5 mol/l NaCl, 6% Triton X-100 versetzt, weitere 30 min bei RT inkubiert und in einer Sorvall RC-5B Zentrifuge (GSA Rotor, 12000 UPM, 15 min) zentrifugiert. Der Überstand wurde verworfen, das Pellet mit 100 ml 50 mmol/l Tris-HCl, pH 7,2, 20 mmol/l EDTA versetzt, 30 min unter Rühren bei 4°C inkubiert und erneut sedimentiert. Der letzte Waschschritt wurde wiederholt. Die gereinigten IBs (1,5-2,0 g Naßgewicht, 25-30% Trockenmasse, 100-150 mg Protease) wurden bis zur Weiterverarbeitung bei -20°C gelagert.

### b) Solubilisierung und Derivatisierung der IBs

Die gereinigten IBs wurden in einer Konzentration von 100 mg IB-Pellet (Naßgewicht)/ml entsprechend 5-10 mg/ml Protein in 6 mol/l Guanidin-HCl, 100 mmol/l Tris-HCl, 20 mmol/l EDTA, 150 mmol/l GSSG und 15 mmol/l GSH, pH 8,0 unter Rühren bei RT in 1-3 Std. gelöst. Anschließend wurde der pH auf pH 5,0 eingestellt und die unlöslichen Bestandteile durch Zentrifugation (Sorvall RC-5B Zentrifuge, SS34 Rotor, 16000 UPM, 10 min) abgetrennt. Der Überstand wurde gegen 100 Vol. 4-6 mol/l Guanidin-HCl pH 5,0 für 24 Std. bei 4°C dialysiert.

### c) Naturierung

Die Naturierung der in 6 mol/l Guanidin-HCl solubilisierten und mit GSSG/GSH derivatisierten Proteasevarianten wurde bei 4°C durch wiederholte (z.B. 5-fache) Zugabe von jeweils 0,5 ml IB-Solubilisat/Derivat zu 50 ml 50 mmol/l Tris-HCl, 0,5 mol/l Arginin, 20 mmol/l CaCl₂, 1 mmol/l EDTA und 0,5 mmol/l Cystein, pH 8,5 im Zeitabstand von 3-5 Std. und anschließende Inkubation für 10-16 Std. bei 4°C bewirkt. Nach Abschluß der Naturierungsreaktion wurden unlösliche Bestandteile durch Filtration mit einem Filtrationsgerät der Firma Satorius (Göttingen, Deutschland) bestückt mit Tiefenfilter K 250 der Firma Seitz (Bad Kreuznach, Deutschland) abgetrennt.

### d) Konzentrierung und Dialyse der Naturierungsansätze

Der klare proteasehaltige Überstand wurde durch Cross-Flow-Filtration in einer Minisette (Membran Typ: Omega 3K) der Firma Filtron (Karlstein, Deutschland) 10-15-fach konzentriert und zur Entfernung von Guanidin-HCl und Arginin gegen 100 Vol. 20 mmol/l Tris-HCl und 50 mmol/l NaCl, pH 8,2 für 12-24 Std. bei 20-25°C dialysiert. Präzipitiertes Protein wurde durch Zentrifugation abgetrennt (Sorvall RC-5B Zentrifuge, SS34 Rotor, 16000 UPM, 20 min) und der klare Überstand mit einer Nalgene® Einmalfiltrationseinheit (Porendurchmesser: 0,2 mm) der Firma Nalge (Rochester, NY, U.S.A.) filtriert.

### Beispiel 8

### Reinigung der naturierten aktiven Trypsin- und FXa Proteasevarianten

Die Aktivierung der Trypsin- und rFX-Proteasevarianten rTRYI-GPK, rTRYI-VGR, rTRYI-GPK-SS und rFX-EGF2-APau-CD durch Autokatalyse erfolgt bereits bei der Naturierung und der anschließenden Konzentrierung und Dialyse des Naturierungsansatzes.

Die aktiven Trypsin- und rFXa Proteasevarianten rTRYI-GPK, rTRYI-VGR, rTRYI-GPK-SS und rFX-EGF2-APau-CD aus den Naturierungsansätzen können bei Bedarf mit chromatographischen Methoden, die dem Fachmann bekannt sind, weiter gereinigt werden.

### Reinigung der Proteasevarianten durch Ionenaustauschchromatographie an Q-Sepharose ff

Der konzentrierte und gegen 20 mmol/l Tris-HCl und 50 mmol/l NaCl, pH 8,2 dialysierte Naturierungsansatz wurde auf eine mit dem gleichen Puffer äquilibrierte Q-Sepharose-ff-Säule (1,5 x 11 cm, V = 20 ml; Beladungskapazität: 10 mg Protein / ml Gel) der Firma Pharmacia Biotech (Freiburg, Deutschland) aufgetragen (2 Säulenvolumen/Stunde, 2 SV/Std.) und so lange mit dem Äquilibrierungspuffer gewaschen, bis die Absorption des Eluats bei 280 nm den Leerwert des Puffers erreichte. Die Elution des gebundenen Materials erfolgte durch einen Gradienten von 50-500 mmol/l NaCl in 20 mmol/l Tris-HCl, pH 8,2 (2 SV/Std.). Die Proteasen wurden bei einer NaCl Konzentration von 100-200 mmol/l eluiert. Die proteasehaltigen Fraktionen wurden durch nicht-reduzierende und reduzierende SDS PAGE identifiziert und der Elutionspeak vereinigt.

### Beispiel 9

### Reinigung der aktiven Proteasevarianten durch Affinitätschromatographie an Benzamidin-Sepharose-CL-6B

Der konzentrierte und gegen 20 mmol/l Tris-HCl und 50 mmol/l NaCl, pH 8,2 dialysierte Naturierungsansatz wurde auf eine mit dem gleichen Puffer äquilibrierte Benzamidin-Sepharose-CL-6B-Säule (1,0 x 10 cm, V = 8 ml; Beladungskapazität: 2-3 mg Protein / ml Gel) der Firma Pharmacia Biotech (Freiburg, Deutschland) aufgetragen (2 SV/Std.) und so lange mit dem Äquilibrierungspuffer gewaschen, bis die Absorption des Eluats bei 280 nm den Leerwert des Puffers erreichte. Die Elution des gebundenen Materials erfolgte durch 10 mmol/l Benzamidin in 20 mmol/l Tris-HCl und 200 mmol/ NaCl, pH 8,2 (2 SV/Std.). Die proteasehaltigen Fraktionen wurden durch nicht-reduzierende und reduzierende SDS PAGE und Aktivitätsbestimmung identifiziert (siehe: Beispiel 10).

Der zur Elution verwendete Serinprotease-Inhibitor Benzamidin wurde durch Dialyse gegen 1 mmol/l HCl (Trypsin) bzw. 50 mmol/l Natriumphosphat-Puffer, pH 6,5 (FXa) entfernt.

### Beispiel 10

### Charakterisierung der gereinigten Proteasevarianten

### a) SDS-PAGE

Sowohl die Oligomeren- und Aggregatbildung durch intermolekulare Disulfidbrückenbildung als auch die Homogenität und Reinheit der naturierten autokatalytisch aktivierten und gereinigten Trypsin- und rFXa Proteasevarianten wurde durch nicht-reduzierende (minus Mercaptoethanol) und reduzierende (plus Mercaptoethanol) SDS PAGE (Laemmli, U.K., Nature 227 (1970) 680-685) untersucht.

### b) Aktivitätsbestimmung, Bestimmung der kinetischen Konstanten Kcat und Km

### Bestimmung der Trypsin Aktivität mit N-Benzoyl-L-argininethylester (BAEE)

Die Aktivitätsbestimmung von Trypsin wurde in Anlehnung an die Vorschrift von Walsh, K.A. und Wilcox, P.E. (Methods Enzymol. XIX (1970) 31-41) in einem Volumen von 1 ml in 63 mmol/l Natriumphosphat-Puffer, pH 7,6 und 0,23 mmol/l N-Benzoyl-L-argininethylester Hydrochlorid (BAEE; Sigma-Aldrich Chemie GmbH, Deisenhofen, Deutschland, Kat.-Nr. B-4500) bei 25°C durchgeführt. Die Reaktion wurde durch Zugabe von 50 µl Trypsin-haltiger Probe (gelöst in 1 mmol/l HCl, 300-600 U/ml) gestartet und die Absorptionsänderung/min (ΔA/min) bei 253 nm bestimmt. 1 BAEE Unit Trypsin ist definiert als die Menge Enzym, die unter den angegebenen Testbedingungen eine Absorptionsänderung (Δ A/min₂₅₃) von 0,0032 bewirkt. Die spezifische Aktivität von gereinigtem humanem Trypsin beträgt unter diesen Testbedingungen ca. 12000 U/mg Protein. Die Aktivität und die kinetischen Konstanten wurden aus der linearen Anfangssteigung gemäß der Michaelis-Menten-Gleichung bestimmt.

### Bestimmung der Trypsin Aktivität mit Chromozym®TH als Substrat

Die Aktivitätsbestimmung von Trypsin wurde in einem Volumen von 1 ml in 50 mmol/l Tris-HCl, 150 mmol/l NaCl, 5 mmol/l CaCl₂, 0,1% PEG 8000, pH 8,0 und 0,1 mmol/l des chromogenen Substrats Chromozym®TH (Tosyl-Gly-Pro-Arg-4-pNA, Kat.-Nr. 838268, Boehringer Mannheim GmbH, Mannheim, Deutschland) bei 25°C durchgeführt. Die Reaktion wurde durch Zugabe von 1-10 µl Trypsin-haltiger Probe (0,4-4 U/ml, gelöst in 1 mmol/l HCl) gestartet und die Absorptionsänderung/min (ΔA/min) bei 405 nm (ε₄₀₅ = 9,75 [mmol⁻¹ x 1 x cm⁻¹]) bestimmt. 1 Chromozym®TH Unit Trypsin ist definiert als die Menge Enzym, die 1 µmol pNitroanilin (pNA) pro min bei 25°C aus dem chromozymen Substrat freisetzt.

### Bestimmung der Faktor Xa Aktivität

Die FXa Aktivitätsbestimmung wurde mit dem chromogenen Substrat Chromozym® X (0,1 mmol/l, N-Methoxycarbonyl-D-Nle-Gly-Arg-pNA, Boehringer Mannheim GmbH, Mannheim, Kat.-Nr. 789763) in einem Volumen von 1 ml bei 25°C in 50 mmol/l Tris-HCl, 150 mmol/l NaCl, 5 mmol/l CaCl₂, 0,1% PEG 8000, pH 8,0, durchgeführt. Die Reaktion wurde durch Zugabe von 1-10 µl rFXa-haltiger Probe (0,4-4 U/ml) gestartet und die Absorptionsänderung/min (ΔA/min) bei 405 nm (ε₄₀₅ = 9,75 [mmol⁻¹ x 1 x cm⁻¹]) gemessen. 1 Unit (U) FXa Aktivität ist definiert als die Menge Enzym, die 1 µmol pNA pro min bei 25°C aus dem chromozymen Substrat freisetzt. Die Aktivität und die kinetischen Konstanten wurden aus der linearen Anfangssteigung gemäß der Michaelis-Menten-Gleichung bestimmt.
Die Ergebnisse zeigt Tabelle 1.

**Tabelle 1**

| **Protease** | **spez. Aktivität** **[U/mg]** | **kcat [1/s]** | **Km [µM]** | **kcat/Km** **[1/µM/s]** |
|---|---|---|---|---|
| Rinder Trypsin¹⁾³⁾ | 230 | 112±2 | 14±1 | 8,0 |
| Schweine Trypsin²⁾³⁾ | 222 | 97±3 | 8,5±0,9 | 11,4 |
| rTRYI-GPK³⁾ | 209 | 95±1 | 13±1 | 7,3 |
| rTRYI-VGR³⁾ | 210 | 95±1 | 13±1 | 7,3 |
| rTRYI-GPK-SS³⁾ | 213 | 99±2 | 19±1 | 5,2 |
| rEGF2-AP-CD⁴⁾ | 206 | --- | --- | --- |
| rEGF2-APau-CD⁴⁾ | 211 | --- | --- | --- |

| | | | | |
|---|---|---|---|---|
| ¹⁾, Rinder Trypsin (Boehringer Mannheim GmbH, Mannheim, Deutschland, Kat.-Nr. 109827) | | | | |
| ²⁾, Schweine Trypsin (Sigma-Aldrich Chemie GmbH, Deisenhofen, Deutschland, Kat.-Nr. T-7418) | | | | |
| ³⁾, Substrat: Chromozym®TH | | | | |
| ⁴⁾, Substrat: Chromozym® X | | | | |

### c) Ermittlung der Trypsin Temperaturstabilität (Tm-Werte)

Die Temperaturstabilität der unterschiedlichen Trypsine wurde durch "differential scanning calorimetry" (DSC) ermittelt. Dazu wurde der Denaturierungspunkt (Tm) der Trypsine in einem definierten Lösungsmittel (1 mmol/l HCl), bei definierter Proteinkonzentration (40 mg/ml) und definierter Aufheizrate (1°C/min) bestimmt (siehe Tabelle 2).

**Tabelle 2**

| **Enzym** | **Tm ( °C ] (DSC)** |
|---|---|
| Rinder Trypsin¹⁾ | 64 |
| Schweine Trypsin²⁾ | 74 |
| rTRYI-GPK | 70 |
| rTRYI-VGR | 70 |
| rTRYI-GPK-SS | 64 |

| | |
|---|---|
| ¹⁾, Rinder Trypsin (Boehringer Mannheim GmbH, Mannheim, Deutschland, Kat.-Nr. 109827) | |
| ²⁾, Schweine Trypsin (Sigma-Aldrich Chemie GmbH, Deisenhofen, Deutschland, Kat.-Nr. T-7418) | |

### d) Bestimmung der Trypsin Restaktivität nach Temperaturbelastung

Zur Ermittlung der Temperaturstabilität wurden die Trypsine in einer Konzentration von 3,5 U/ml in 50 mol/l Tris-HCl, 150 mmol/l NaCl und 5 mmol/l CaCl₂, pH 8,0, bei 45°C inkubiert und nach 3, 6 und 29 Std. die Trypsin Restaktivität mit Chromozym®TH als Substrat, wie in Beispiel 10 b beschrieben, bestimmt. Die Ergebnisse zeigt Tabelle 3.

**Tabelle 3**

| **Enzym** | **Trypsin Restaktivität [ % ]** | | |
|---|---|---|---|
| | **3 [ Std. ]** | **6 [ Std. ]** | **29 [ Std. ]** |
| Rinder Trypsin¹⁾ | 80 | 69 | 22 |
| Schweine Trypsin²⁾ | 96 | 98 | 71 |
| rTRYI-GPK | 96 | 97 | 96 |
| rTRYI-VGR | 97 | 96 | 97 |
| rTRYI-GPK-SS | 90 | 87 | 84 |

| | | | |
|---|---|---|---|
| ¹⁾, Rinder Trypsin (Boehringer Mannheim GmbH, Mannheim, Deutschland, Kat.-Nr. 109827) | | | |
| ²⁾, Schweine Trypsin (Sigma-Aldrich Chemie GmbH, Deisenhofen, Deutschland, Kat.-Nr. T-7418) | | | |

### Referenzliste

Bharadwaj, D. et al., J. Biol. Chem. 270 (1995) 6537-6542
Blow, D.M.: Acc. Chem. Res. 9 (1976) 145-152
Brewer, S.J. und Sassenfeld, H.M., Trends in Biotechnology 3 (1985) 119-122
Brinkmann, U. et al., Gene 85 (1989) 109-114
Bristow, A.F.: The current status of therapeutic peptides and proteins. In: Hider, R.C.; Barlow, D., eds., Polypeptides and Protein Drugs. Production, Characterization and Formulation. London, Ellis Horwood, pp. 54-69 (1991)
Carter, P.: Site-specific proteolysis of fusion proteins. In: Ladisch, M.R.; Willson, R.C.; Painton, C.C.; Builder, S.E. eds., Protein Purification: From Molecular Mechanisms to Large-Scale Processes. ACS Symposium Series No. 427, American Chemical Society, pp. 181-193 (1990)
Creighton, T.E., Progress Biophys. Molec. Biol. 33 (1978) 231 - 291
Davie, E.W. et al., Biochem. 30 (1991) 10363-10370
DiBella, E.E. et al., J. Biol. Chem. 270 (1995) 163-169
Emi, M. et al., Gene 41 (1986) 305-310
EP-A 0 597 681
EP-A 0 089 626
EP-A 0 189 998
EP-A 0 367 302
EP-B 0 114 506
EP-B 0 219 874
EP-B 0241 022
EP-B 0 393 725
EP-A 0 408 764
EP-A 0 467 839
EP-B 0 513 073
Flaschel, E. und Friehs, K., Biotech. Adv. 11 (1993) 31-78
Forsberg, G. et al., J. Prot. Chem. 10 (1991) 517-526
Forsberg, G. et al., J. Prot. Chem. 11 (1992) 201-211
Furie, B. und Furie, B.C., Cell 53 (1988) 505- 518
Graf, E. et al. (Biochem. 26 (1987) 2616-2623
Graf, E. et al. , Proc. Natl. Acad. Sci. USA 85 (1988) 4961-4965
Grodberg, J. und Dunn, J.J., J. Bacteriol. 170 (1988) 1245-1253
Guy, O. et al., Biochem. 17 (1978) 1669-1675
Hedstrom, L. et al., Science 255 (1992) 1249-1253
Higaki, J.N. et al., Biochem. 28 (1989) 9256-9263
Jonasson, P. et al., Eur. J. Biochem. 236 (1996) 656-661
Laemmli, U.K., Nature 227 (1970) 680-685
Mann, K. G. et al., Blood 76 (1990) 1-16
Marston, F.A.O., Biochem. J. 240 (1986) 1-12
Medved, L.V. et al., J. Biol. Chem. 270 (1995) 13652-13659
Meyer-Ingold, W.,TIBTECH 11(1993) 387-392
Miller, C.G. et al., Proc. Natl. Acad. Sci. USA 84 (1987) 2718-2722
Mitraki, A. und King, J., Bio/Technology 7 (1989) 690 - 697
Mullis, K.B. und Faloona, F.A., Methods Enzymol. 155 (1987) 355-350
Nilsson, B. et al., Methods Enzymol. 185 (1991) 3-16
Parks, T.D. et al., Anal. Biochem. 216 (1994) 413-417
Perona, J.J. und Craik, C.S., Protein Science 4 (1995) 337-360
Pohlner, J. et al., Nature 325 (1987) 458-462
Polgar, L.: Structure and function of serine proteases. In: Mechanisms of protein action. Boca Raton, Florida, CRC Press, chapter 3 (1989)
Rote Liste, 1997
Sambrook, J.; Fritsch, E.F.; Maniatis,T.: Molecular cloning: A laboratory manual. Cold Spring Harbor Press, Cold Spring Harbor, New York, (1989)
Sassenfeld, H.M., Trends Biotechnol. 8 (1990) 88-93
Schein, C.H., Bio/Technology 8 (1990) 308 - 317
Stryer, L., Biochemistry, Freeman and Company, W.H., San Francisco, 1975, Seiten 24 - 30
Takeya, H. et al., J. Biol. Chem. 267 (1992) 14109-14117
The United States Pharmacopeia, The National Formulary, USP23-NF 18, 1995
US Patent 4,634,671
US Patent 5,432,062
Vasquez, J.R. et al. (J. Cell. Biochem. 39 (1989) 265-276
Walsh, K.A. und Wilcox, P.E., Methods Enzymol. XIX (1970) 31-41
Wang, E.A., TIBTECH 11 (1993) 379-383
Willett, W.S. et al., Biochem. 34 (1995) 2172-2180
WO 93/09144
WO 96/17942
WO 96/20724
WO 97/03027
WO 97/11186
WO 97/18314
WO 97/00316
Yee, L. und Blanch, H.W., Biotechnol. Bioeng. 41 (1993) 781-790

## Patentansprüche

1. Verfahren zur rekombinanten Herstellung einer Serinprotease, **gekennzeichnet durch**:
a) Transformation einer prokaryontischen Wirtszelle mit einer rekombinanten Nukleinsäure, welche für eine zymogene Vorstufe der genannten Serinprotease codiert, wobei in der zymogenen Vorstufe eine natürlich vorkommende nicht-autokatalytische Spaltstelle **durch** eine autokatalytische Spaltstelle ersetzt ist, die von der aktiven Form der Protease erkannt wird und dabei die zymogene Vorstufe in die aktive Form spaltet,
b) Kultivierung der Wirtszelle und Expression der Nukleinsäure,
c) Isolierung der im Cytoplasma entstandenen und die zymogene Vorstufe enthaltenden inclusion bodies,
d) Naturierung der zymogenen Vorstufe und
e) autokatalytische Spaltung der zymogenen Vorstufe in die aktive Form der Serinprotease.

2. Verfahren nach Anspruch 1, **dadurch gekennzeichnet, dass** die Serinprotease Trypsin, Thrombin, Faktor Xa oder Lysyl-Endoproteinase ist.

3. Autokatalytisch spaltbare zymogene Vorstufe einer Serinprotease, wobei in der zymogenen Vorstufe eine natürlich vorkommende nicht-autokatalytische Spaltstelle durch eine autokatalytische Spaltstelle ersetzt ist.

4. Verfahren zur rekombinanten Herstellung von Trypsin, **dadurch gekennzeichnet, dass** eine prokaryontische oder eukaryontische Wirtszelle mit einer Nukleinsäure transformiert wird, welche für ein Trypsin der Aminosäuresequenz SEQ ID NO:12 codiert, die Nukleinsäure exprimiert und das dabei entstandene Protein isoliert wird.

5. Verfahren nach Anspruch 1, **dadurch gekennzeichnet, dass** das Verhältnis der proteolytischen Aktivität der zymogenen Vorstufe und der aktiven Serinprotease 1 : 5 oder weniger beträgt.

6. Präparation einer autokatalytisch spaltbaren zymogenen Vorstufe einer Serinprotease, wobei in der zymogenen Vorstufe eine natürlich vorkommende nicht-autokatalytische Spaltstelle durch eine autokatalytische Spaltstelle ersetzt ist, **dadurch gekennzeichnet, dass** das Protein inaktiv und denaturiert in der Form von inclusion bodies in einer prokaryontischen Zelle vorliegt.

7. Präparation von inclusion bodies, welche eine autokatalytisch spaltbare zymogene . Vorstufe einer Serinprotease enthalten, wobei in der zymogenen Vorstufe eine natürlich vorkommende nicht-autokatalytische Spaltstelle durch eine autokatalytische Spaltstelle ersetzt ist.

## Claims

1. Process for the recombinant production of a serine protease, **characterized by**:
a) transforming a prokaryotic host cell with a recombinant nucleic acid which codes for a zymogenic precursor of the said serine protease wherein a naturally occurring non-autocatalytic cleavage site is replaced in the zymogenic precursor by an autocatalytic cleavage site which is recognized by the active form of the protease and thus cleaves the zymogenic precursor into the active form,
b) culturing the host cell and expressing the nucleic acid,
c) isolating the inclusion bodies that are formed in the cytoplasm and contain the zymogenic precursor,
d) renaturing the zymogenic precursor and
e) autocatalytically cleaving the zymogenic precursor into the active form of the serine protease.

2. Process as claimed in claim 1, **characterized in that** the serine protease is trypsin, thrombin, factor Xa or lysyl endoproteinase.

3. Autocatalytically cleavable zymogenic precursor of a serine protease wherein a naturally occurring non-autocatalytic cleavage site is replaced in the zymogenic precursor by an autocatalytic cleavage site.

4. Process for the recombinant production of trypsin, **characterized in that** a prokaryotic or eukaryotic host cell is transformed with a nucleic acid that codes for a trypsin having the amino acid sequence SEQ ID NO:12, the nucleic acid is expressed and the protein that is formed in this process is isolated.

5. Process as claimed in claim 1, **characterized in that** the ratio of the proteolytic activity of the zymogenic precursor to that of the active protease is 1:5 or less.

6. Preparation of an autocatalytically cleavable zymogenic precursor of a serine protease in which a naturally occurring non-autocatalytic cleavage site is replaced in the zymogenic precursor by an autocatalytic cleavage site, **characterized in that** the protein is present in an inactive and denatured form as inclusion bodies in a prokaryotic cell.

7. Preparation of inclusion bodies which contain an autocatalytically cleavable zymogenic precursor of a serine protease in which a naturally occurring non-autocatalytic cleavage site is replaced in the zymogenic precursor by an autocatalytic cleavage site.

## Revendications

1. Procédé pour la production par recombinaison d'une sérine-protéase, **caractérisé par**
a) la transformation d'une cellule hôte procaryote avec un acide nucléique recombinant, qui code pour un précurseur zymogène de la sérine-protéase mentionnée, un site de clivage non auto-catalytique présent à l'état naturel dans le précurseur zymogène étant remplacé par un site de clivage auto-catalytique, qui est reconnu par la forme active de la p rotéase et qui clive le précurseur zymogène dans la forme active,
b) la culture de la cellule hôte et l'expression de l'acide nucléique,
c) l'isolement des corps d'inclusion formés dans le cytoplasme et contenant le précurseur zymogène,
d) la naturation du précurseur zymogène et
e) le clivage auto-catalytique du précurseur zymogène en la forme active de la sérine-protéase.

2. Procédé selon la revendication 1, **caractérisé en ce que** la sérine-protéase est la trypsine, la thrombine, le facteur Xa ou la lysyl-endoprotéinase.

3. Précurseur zymogène clivable par voie auto-catalytique d'une sérine-protéase, un site de clivage non auto-catalytique présent à l'état naturel dans le précurseur zymogène étant remplacé par un site de clivage auto-catalytique.

4. Procédé pour la production par recombinaison de trypsine, **caractérisé en ce qu'**une cellule hôte procaryote ou eucaryote est transformée avec un acide nucléique qui code pour une trypsine présentant la séquence d'acides aminés SEQ ID NO:12, exprime l'acide nucléique et la protéine formée est isolée.

5. Procédé selon la revendication 1, **caractérisé en ce que** le rapport de l'activité protéolytique du précurseur zymogène à la sérine-protéase active est de 1:5 ou moins.

6. Préparation d'un précurseur zymogène clivable par voie auto-catalytique d'une sérine-protéase, un site de clivage non auto-catalytique présent à l'état naturel dans le précurseur zymogène étant remplacé par un site de clivage auto-catalytique, **caractérisée en ce que** la protéine inactive et dénaturée se trouve sous la forme de corps d'inclusion dans une cellule procaryote.

7. Préparation de corps d'inclusion, qui contiennent un précurseur zymogène clivable par voie auto-catalytique d'une sérine-protéase, un site de clivage non auto-catalytique présent à l'état naturel dans le précurseur zymogène étant remplacé par un site de clivage auto-catalytique.


### SEQUENZPROTOKOLL

(1) ALLGEMEINE ANGABEN:
   (i) ANMELDER:
      (A) NAME: BOEHRINGER MANNHEIM GMBH
      (B) STRASSE: Sandhofer Str. 116
      (C) ORT: Mannheim
      (E) LAND: Germany
      (F) POSTLEITZAHL: D-68305
      (G) TELEFON: 08856/60-3446
      (H) TELEFAX: 08856/60-3451
   (ii) BEZEICHNUNG DER ERFINDUNG: Autokatalytisch aktivierbare zymogene Vorstufen von Proteasen und deren Verwendung
   (iii) ANZAHL DER SEQUENZEN: 12
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
(2) ANGABEN ZU SEQ ID NO: 1:
   (i) SEQUENZKENNZEICHEN:
      (A) LÄNGE: 35 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N1""
   (xi) SEQUENZBESCHREIBUNG: SEQ ID NO: 1:
   AAAAAACCAT GGATGATGAT GACAAGATCG TTGGG 35
(2) ANGABEN ZU SEQ ID NO: 2:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N2""
   (xi) SEQUENZBESCHREIBUNG: SEQ ID NO: 2:
   AAAAAAAAGC TTCATTAGCT ATTGGCAGCT ATGGTGTTC 39
(2) ANGABEN ZU SEQ ID NO: 3:
   (i) SEQUENZKENNZEICHEN:
      (A) LÄNGE: 58 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N3""
   (xi) SEQUENZBESCHREIBUNG: SEQ ID NO: 3:
   AAAAAAACCA TGGGTCCGAA AATCGTTGGT GGTTACAATT GTGAGGAGAA TTCTGTCC 58
(2) ANGABEN ZU SEQ ID NO: 4:
   (i) SEQUENZKENNZEICHEN:
      (A) LÄNGE: 58 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N4""
   (xi) SEQUENZBESCHREIBUNG: SEQ ID NO: 4:
   AAAAAAACCA TGGTTGGTCG TATCGTTGGT GGTTACAATT GTGAGGAGAA TTCTGTCC 58
(2) ANGABEN ZU SEQ ID NO: 5:
   (i) SEQUENZKENNZEICHEN:
      (A) LÄNGE: 77 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N5""
   (xi) SEQUENZBESCHREIBUNG: SEQ ID NO: 5:
   AAAAAAGTGC AGTAATCAAC GCCCGCGTGT CCACCATCTC TCTGCCCACC GCCTGCGCTG 60
   CCACTGGTAC GAAGTGC 77
(2) ANGABEN ZU SEQ ID NO: 6:
   (i) SEQUENZKENNZEICHEN:
      (A) LÄNGE: 80 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N6""
   (xi) SEQUENZBESCHREIBUNG: SEQ ID NO: 6:
   AAAAAAAAGC TTCATTAGCT ATTGGCAGCT ATGGTGTTCT TAATCCATTT CACATAGTTG 60
   CAGACCTTGG TGTAGACTCC 80
(2) ANGABEN ZU SEQ ID NO: 7:
   (i) SEQUENZKENNZEICHEN:
      (A) LÄNGE: 29 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N7""
   (xi) SEQUENZBESCHREIBUNG: SEQ ID NO: 7:
   AAAAAAAGGC CTGCATTCCC ACAGGGCCC 29
(2) ANGABEN ZU SEQ ID NO: 8:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N8""
   (xi) SEQUENZBESCHREIBUNG: SEQ ID NO: 8:
   AAAAAACCAC GCTCTGGCTG CGTCTGGTTG AAGTCAAG 38
(2) ANGABEN ZU SEQ ID NO: 9:
   (i) SEQUENZKENNZEICHEN:
      (A) LÄNGE: 60 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N9""
   (xi) SEQUENZBESCHREIBUNG: SEQ ID NO: 9:
   AAAAAACCAG AGCGTGGCGA CAACATCGAC GGTAGGATCG TGGGAGGCCA GGAATGCAAG 60
(2) ANGABEN ZU SEQ ID NO: 10:
   (i) SEQUENZKENNZEICHEN:
      (A) LÄNGE: 41 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Primer "N10""
   (xi) SEQUENZBESCHREIBUNG: SEQ ID NO: 10:
   AAAAAAAAGC TTCATTACTT GGCCTTGGGC AAGCCCCTGG T 41
(2) ANGABEN ZU SEQ ID NO: 11:
   (i) SEQUENZKENNZEICHEN:
      (A) LÄNGE: 52 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 11:
   Ser Val Ala Gln Ala Thr Ser Ser Ser Gly Glu Ala Pro Asp Ser Ile
   1               5                   10                  15
   Thr Trp Lys Pro Tyr Asp Ala Ala Asp Leu Asp Pro Thr Glu Asn Pro
               20                  25                  30
   Phe Asp Leu Leu Asp Phe Asn Gln Thr Gln Pro Glu Arg Gly Asp Asn
           35                  40                  45
   Asn Leu Thr Arg
       50
(2) ANGABEN ZU SEQ ID NO: 12:
   (i) SEQUENZKENNZEICHEN:
      (A) LÄNGE: 701 Basenpaare
      (B) ART: Nucleotid
      (C) STRANGFORM: Einzelstrang
      (D) TOPOLOGIE: linear
   (ii) ART DES MOLEKÜLS: cDNA
   (xi) SEQUENZBESCHREIBUNG: SEQ ID NO: 12:
   ATGGATGATG ATGACAAGAT CGTTGGGGGC TACAACTGTG AGGAGAATTC TGTCCCCTAC    60
   CAGGTGTCCC TGAATTCTGG CTACCACTTC TGTGGTGGCT CCCTCATCAA CGAACAGTGG   120
   GTGGTATCAG CAGGCCACTG CTACAAGTCC CGCATCCAGG TGAGACTGGG AGAGCACAAC   180
   ATCGAAGTCC TGGAGGGGAA TGAGCAGTTC ATCAATGCAG CCAAGATCAT CCGCCACCCC   240
   CAATACGACA GGAAGACTCT GAACAATGAC ATCATGTTAA TCAAGCTCTC CTCACGTGCA   300
   GTAATCAACG CCCGCGTGTC CACCATCTCT CTGCCCACCG CCCCTCCAGC CACTGGCACG   360
   AAGTGCCTCA TCTCTGGCTG GGGCAACACT GCGAGCTCTG GCGCCGACTA CCCAGACGAG   420
   CTGCAGTGCC TGGATGCTCC TGTGCTGAGC CAGGCTAAGT GTGAAGCCTC CTACCCTGGA   480
   AAGATTACCA GCAACATGTT CTGTGTGGGC TTCCTTGAGG GAGGCAAGGA TTCATGTCAG   540
   GGTGATTCTG GTGGCCCTGT GGTCTGCAAT GGACAGCTCC AAGGAGTTGT CTCCTGGGGT   600
   GATGGCTGTG CCCAGAAGAA CAAGCCTGGA GTCTACACCA AGGTCTACAA CTACGTGAAA   660
   TGGATTAAGA ACACCATAGC TGCCAATAGC TAATGAAGCT T                       701